# EUROPEAN PATENT APPLICATION

(11) **EP 3 848 001 A1**
(43) Date of publication of application: **14.07.2021**
(21) Application number: 21150549.0
(22) Date of filing: 07.01.2021
(51) Int. Cl.: A61F 2/24

(54) **MULTI-COMPONENT VALVE IMPLANT AND DELIVERY METHODS**

(30) Priority: 08.01.2020 US 202062958374 P
(71) Applicant: Cephea Valve Technologies, Inc., Santa Clara, CA 95054 (US)
(72) Inventor: HAYNES, Evelyn, Soquel, CA 95073 (US)
(74) Representative: J A Kemp LLP

(57) **Abstract**

The present disclosure provides systems and methods for a replacement mitral valve. The replacement mitral valve may include a ventricular portion and a separate atrial portion tethered to the ventricular portion. When in the delivery sheath, the ventricular and atrial portions may be in a collapsed configuration. The ventricular portion and atrial portion may be axially offset within the delivery sheath thereby reducing the diameter of the replacement valve when in the collapsed configuration and the diameter necessary for the delivery sheath. Upon exiting the delivery sheath, the ventricular and atrial portions may expand. Once in the expanded configuration, the ventricular and atrial portions may be cinched together via an anchoring mechanism.

## Description

### BACKGROUND OF THE DISCLOSURE

Heart valve disease is a significant cause of morbidity and mortality. A primary treatment of this disease is valve replacement. One form of replacement device is a bioprosthetic valve. Collapsing these valves to a smaller size or into a delivery system enables less invasive delivery approaches compared to conventional open-heart surgery. Collapsing the implant to a smaller size and using a smaller delivery system minimizes the access site size and reduces the number of potential periprocedural complications.

The size that an implant can be collapsed to is limited by the volume of material used in the implant, the strength and shape of that material, and the need to function after re-expansion. Using multiple steps and/or multiple delivery system devices increases the time and complexity of a procedure.

### BRIEF SUMMARY

One aspect of the disclosure is a replacement cardiac valve comprising an atrial portion, a ventricular portion, and a plurality of links. The atrial portion may be configured to expand from a collapsed configuration to an expanded configuration. The ventricular portion may be separate from the atrial portion and configured to expand from a collapsed configuration to an expanded configuration. The ventricular portion may have a first plurality of anchoring points. Each of the plurality of links may be coupled to a respective one of the first plurality of anchoring points. At least one of the atrial portion or the ventricular portion may be moveable with respect to the plurality of links to adjust a distance between the atrial portion and the ventricular portion when the atrial portion and the ventricular portion are each in the expanded configuration.

Another aspect of the disclosure is a method of delivering a replacement cardiac valve having a collapsed configuration within a delivery sheath of a delivery device. The method comprises delivering a ventricular portion having a first plurality anchoring points from the collapsed configuration in the delivery sheath, wherein the ventricular portion expands inside a first heart chamber on a first side of the mitral valve annulus, the ventricular portion having a first plurality of anchoring points. The method further comprises delivering an atrial portion from the collapsed configuration in the delivery sheath to a second heart chamber on a second side of the mitral valve annulus, wherein the atrial portion expands on the second side. The method further comprises adjusting a distance between the ventricular portion and the atrial portion, wherein the adjusting comprises moving the atrial portion with respect to a plurality of links coupled to the first plurality of anchoring points.

In the above method the adjusting a distance between the ventricular portion and the atrial portion further may comprise moving an anchoring mechanism having a flexible locking mechanism distally, wherein the anchoring mechanism is located proximally to a luminal surface of the atrial anchor.

In the above method each of the plurality of links may further include a flexible locking mechanism and a plurality of locking features, each flexible locking mechanism configured to engage the plurality of locking features.

In the above method moving the atrial portion with respect to a plurality of links may further comprise moving the flexible locking mechanism distally over the plurality of locking features.

In the above method the atrial portion may include a second plurality of anchoring points, each of the plurality of links extending through a respective one of the second plurality of anchoring points. In the above method, when the atrial portion and the ventricular portion are in the collapsed configuration the atrial portion may be a first distance from the ventricular portion, and when the atrial portion and the ventricular portion are in the expanded configuration the method may further include moving the flexible locking mechanism such that the atrial portion is a second distance from the ventricular portion, the second distance being smaller than the first distance.

### BRIEF DESCRIPTION OF THE DRAWINGS

Figure 1A illustrates a perspective view of an example replacement valve in an expanded configuration according to aspects of the disclosure.
Figure 1B illustrates a perspective view of a ventricular portion of the replacement valve in an expanded configuration according to aspects of the disclosure.
Figure 1C illustrates a top view of the ventricular portion of the replacement valve in an expanded configuration according to aspects of the disclosure.
Figure 1D illustrates a perspective view of an atrial portion of the replacement valve in an expanded configuration according to aspects of the disclosure
Figure 1E illustrates a top view of the atrial portion of the replacement valve in an expanded configuration according to aspects of the disclosure.
Figure 2A illustrates a cross-section of an example replacement valve in a collapsed configuration within a delivery sheath according to aspects of the disclosure.
Figure 2B illustrates a cross-section of the replacement valve of Figure 2A in an expanded configuration according to aspects of the disclosure.
Figure 3A illustrates a cross-section of another example replacement valve in a collapsed configuration within a delivery sheath according to aspects of the disclosure.
Figure 3B illustrates a cross-section of a ventricular portion of the replacement valve in Figure 3A exiting the delivery sheath according to aspects of the disclosure.
Figure 3C illustrates a cross-section of an atrial portion of the replacement valve in Figure 3A exiting the delivery sheath according to aspects of the disclosure.
Figures 3D and 3E illustrate cross-sections of the replacement valve of Figure 3A being anchored to different native mitral valve annuli according to aspects of the disclosure.
Figure 4A illustrates a cross-section of another example replacement valve in a collapsed configuration within a delivery sheath according to aspects of the disclosure.
Figure 4B illustrates a cross-section of the replacement valve of Figure 4A in an expanded configuration according to aspects of the disclosure.
Figure 5A-5D illustrate an example anchoring mechanism according to aspects of the disclosure.
Figure 6A-6D illustrate another example anchoring mechanism according to aspects of the disclosure.
Figure 7 is a flow diagram illustrating an example method according to aspects of the disclosure.

### DETAILED DESCRIPTION

The present disclosure provides for a collapsible cardiac valve implant separated into at least two portions or regions. The disclosure further provides for a single delivery device deployment of the collapsible cardiac valve implant and assembly. The portions of the collapsible cardiac valve implant may collapse into a delivery system such that the portions may be axially offset from their final position to reduce the overall profile of the collapsible cardiac valve implant by reducing or eliminating radial overlap of the two portions when collapsed. Reducing this overlap may allow the portions to collapse into a smaller system than would be possible if the portions were collapsed in the delivery system while in their final position. The separate portions may be deployed in a sequence, and then cinched together before their final release from the delivery device. Deploying the multiple implant portions from one device may help maintain a streamlined procedure for the device user.

Assembling the two portions during delivery can enable patient specific tuning of the final assembly. This may allow for accommodation of different anatomies or disease states. For example, tuned attachment may better conform the prosthetic implant to regions of calcification or thickened native leaflet material.

In the context of replacement mitral valves, the "distal" end of the replacement valve refers to the end of the replacement valve that is to be positioned on the ventricular side of the annulus, while "proximal" end refers to the end of the replacement valve that is to be positioned on the atrial side of the annulus. "Distally" in the context of delivery can be used to refer to a location closer to the left ventricle than the left atrium, while "proximally" is generally used to refer to a location closer to the left atrium than the left ventricle.

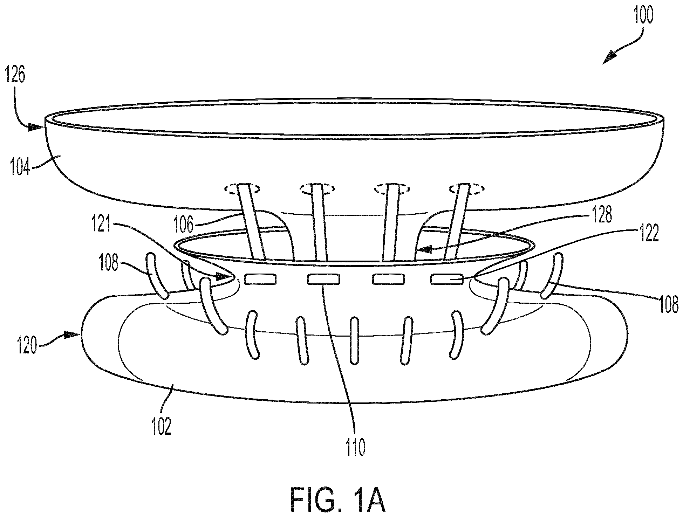

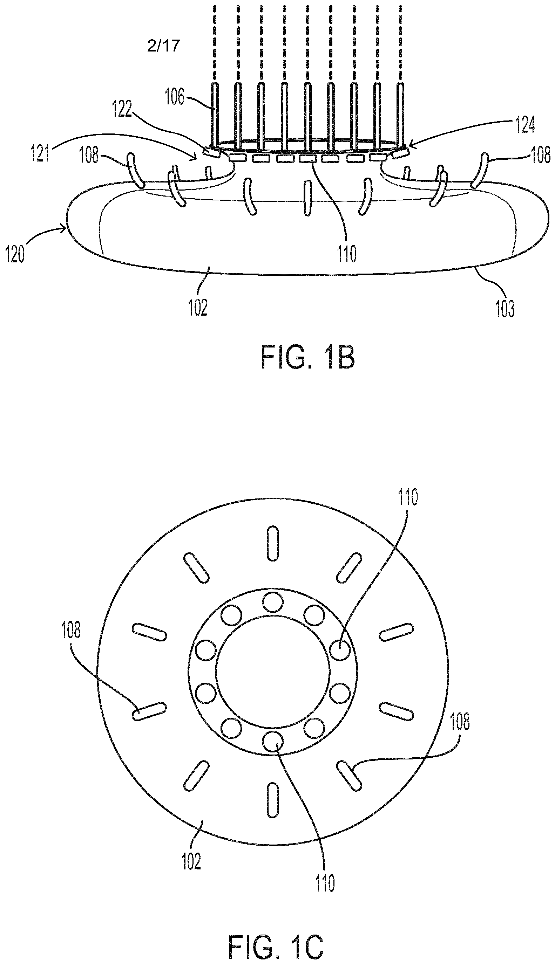

Figure 1A illustrates an example of a replacement valve for replacing a heart valve, and particularly atrioventricular valves such as the mitral valve or the tricuspid valve. The replacement valve 100 includes at least two separate portions or anchors, with a first portion being a ventricular portion 102 and a second portion being an atrial portion 104. The ventricular portion 102 is configured to be positioned on a ventricular side of the native valve annulus, and the atrial portion 104 is configured to be positioned on an atrial side of the native valve annulus. A plurality of links 106 may tether or connect the ventricular portion 102 to the atrial portion 104 when the replacement valve 100 is in an assembled condition or a final implanted condition. Examples of suitable structures for the links are described in greater detail below. In the expanded configuration shown in Figure 1A, ventricular portion 102 and atrial portion 104 extend radially outward from a central longitudinal axis of the replacement valve 100, and may be considered to flare outwardly relative to the central longitudinal axis.

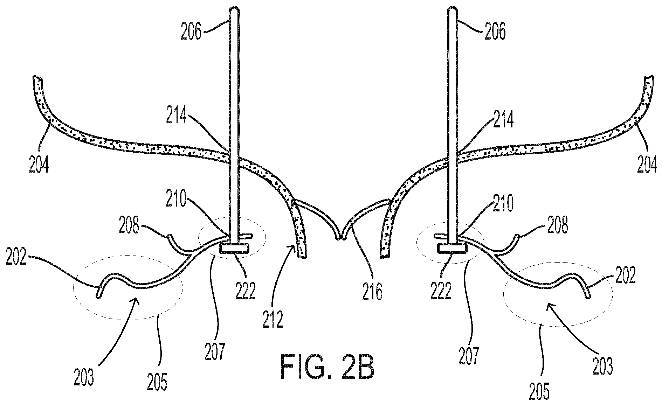

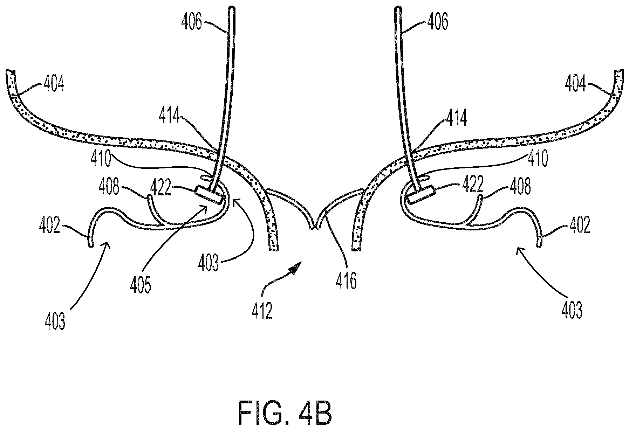

Figures 1B-1C illustrate one example of the ventricular portion 102. The ventricular portion 102 may include a stent, a cuff, a plurality of anchoring points, and a plurality of anchoring features including barbs. The ventricular portion 102 may be configured and adapted to be disposed on a ventricular side of the native valve annulus. The ventricular portion 102 may include a stent or other support structure that includes or is formed of a plurality of diamond shaped cells, although other suitable shapes, such as triangular, quadrilateral, or polygonal may be appropriate. In some examples, the ventricular portion 102 may be formed as a braided mesh, as a unitary stent, or a combination thereof. According to one example, the ventricular portion 102 may be laser cut from a tube of nitinol and heat set to a desired shape so that the ventricular portion 102 is collapsible for delivery, and re-expandable to the set-shape during deployment. The ventricular portion 102 may be partially or entirely covered by a cuff or skirt, on the luminal and/or abluminal surface of the ventricular portion 102. The skirt may be formed of any suitable material, including biomaterials such as bovine pericardium, biocompatible polymers such as ultra-high molecular weight polyethylene, or combinations thereof. The ventricular portion 102 may have contours or curves that may be used to modify stiffness of the ventricular portion. For example, as shown in Figures 2B and 4B, the ventricular portion may have contours 203, 403. The ventricular portion may flex radially inward such that the outermost regions 205 may flex more than the portions 207 of the ventricular portion 202 closest to the ventricular anchoring points. The outermost region 205 and portion 207 of the ventricular portion 202 closest to the ventricular anchoring points are indicated by dashed lines, as shown in Figure 2B. The areas depicted as the outermost region 205 and portion 207 are merely examples and may range in size and shape depending on the size and shape of the ventricular portion 202. Thus, the example shown in Figure 2B is not meant to be limiting. In some examples, the outermost regions 205 may include contours 203, 403. In yet another example, the outermost region 205 may be contours 203, 403. Contours 203, 403 may increase or decrease the amount of flex the outermost regions 205 experience. The ventricular portion, as a whole, may be able to flex radially inward. The outermost region 205 may flex more due to their distance from the center of the replacement valve. The contours 203, 403 may determine how much of the motion or flex of the ventricular portion is shifted radially inward. The counter and/or the amount of metal at the outside of the profile may impact how much the contours 203, 403 curl or flex as pressure is applied. The contours of the ventricular portion 102 may be used to modify the ventricular portion 102 to better fit the anatomy of the mitral valve. The outermost tip 103 of the ventricular portion 102 may be curved inwardly. The outermost tip 103 may be the distal most end or substantially distal most end of the ventricular portion 102. According to some examples, the curve of the outermost tip 103 may be curved inward toward a central longitudinal axis. The inward curve, for example, may extend along an arc equivalent to a quarter of a circle, or 90 degrees. In other examples, the inward curve may extend along an arc equivalent to a semi-circle, or 180 degrees. However, the inward curve may extend along any degree of an arc, such as 22 degrees, 48.3 degrees, 104 degrees, etc. Thus, the examples provided herein are not meant to be limiting. The amount of curvature may be dependent on the thickness of the ventricular portion. For example, a less thick ventricular portion may curve more than a thicker ventricular portion. The ventricular portion 102 may be curved inwardly to provide for atraumatic anatomical contact with the native mitral valve orifice. In some examples, the ventricular portion 102 may be curved inwardly to provide for atraumatic anatomical contact with the sub-valvular apparatus.

As shown in Figures 1A and 1B, the ventricular portion 102 may have three regions, a first region 120 with a disc shape at an outflow end, a central region 121 adjacent the first region 120, and a third flared region 124 adjacent the central region 121. The disc shape of the ventricular portion 102 may be configured to fit into and conform against the ventricular side of the native valve annulus when in the expanded configuration. The disc shape of the first region 120 may also help resist migration of the replacement valve 100 toward the atrium when the replacement valve 100 is implanted.

The first region 120, having the disc shape, may have a first diameter that is the largest diameter of the ventricular portion 102 when in the expanded configuration. The central region 121 may have the smallest diameter of the ventricular portion 102 when in the expanded configuration. The diameter of the disc shape region 120 may taper to the smaller diameter of the central region 121. The flared region 124 may be flared outwardly relative to the central longitudinal axis of the replacement valve 100 when in the expanded configuration. The flared region 124 may have a diameter that is larger than the central region 121 but smaller than the diameter of the disc-shaped region 120 when in the expanded configuration. The central region 121 may taper to the larger flared region 124.

The ventricular portion 102 may include a plurality of retention features 108. The retention features 108 may be, for example, barbs. The retention features 108 may extend radially outwardly from surface of the ventricular portion 102 that will abut the surface of the native valve annulus facing the ventricle. For example, the retention features 108 may extend from an abluminal surface of the ventricular portion 102. The retention features 108 may secure the ventricular portion 102 to a location in the ventricle, which may help resist migration of the replacement valve toward the atrium when the replacement valve 100 is implanted. In some examples, the retention features 108 may be configured to engage with the ventricular side of a native mitral valve annulus when the ventricular portion 102 is in the expanded configuration. The curvature of the retention features 108, location of the retention features 108, and/or length of the retention features may impact the amount of tissue each of the retention features engage. In some examples, the curvature of the retention features 108, location of the retention features 108, and/or length of the retention features may impact how hard it may be to pull the retention features 108 through or out of the tissue.

The ventricular portion 102 may further include a plurality of ventricular anchoring points 110. The ventricular anchoring points 110 may be a plurality of holes for configured to receive links 106. The ventricular anchoring points 110 may be located at or near a proximal end of the ventricular portion 102, for example at the flared region 124. The proximal end of the ventricular portion 102 refers to the end of the ventricular portion 102 that is closest to the native valve annulus when the replacement valve 100 is positioned within the native valve annulus. In some examples, the ventricular anchoring points 110 may be located in the central region 121. In yet another example, the ventricular anchoring points 110 may be located at any location between the first/disc-shaped region 120 and the flared region 124. Thus, the location of the ventricular anchoring points 110 shown in Figure 1A is merely one example and is not meant to be limiting.

The plurality of ventricular anchoring points 110 may be configured and adapted to receive the plurality of links 106. For example, each link 106 may be coupled to a ventricular anchoring point 110. The links 106 may be integral with the ventricular anchoring points 110 such that the links 106 are integral with the ventricular portion 102. According to some examples, each link 106 may include a stop 122 that is larger than ventricular anchoring point 110. In such an example, each link 106 may be inserted into and pulled through the ventricular anchoring points 110 until stop 122 abuts the ventricular potion 102. The links 106 may extend proximally away from the distal end of ventricular portion 102. Examples of suitable structures for links 106 and stops 122 are described in greater detail below.

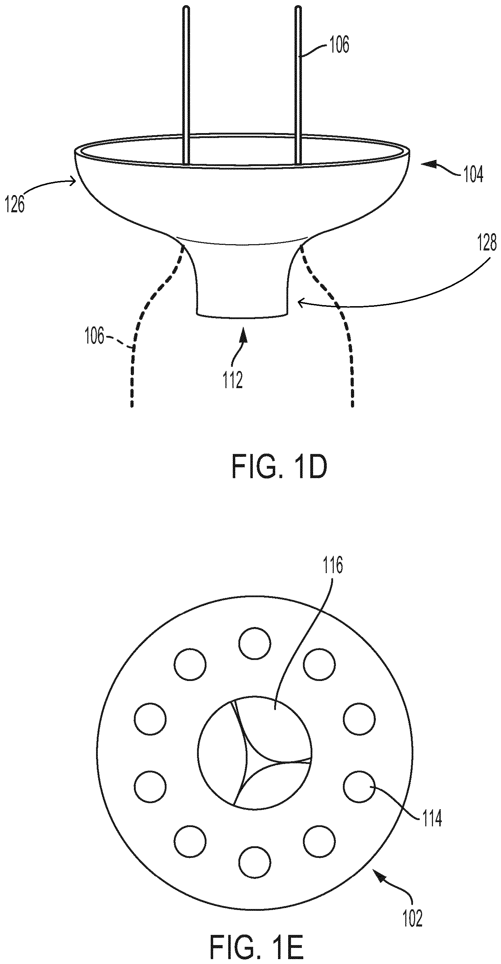

Figures 1D and 1E illustrate one example of the atrial portion 104 of the replacement valve 100. The atrial portion 104 may be configured and adapted to be disposed on an atrial side of the native valve annulus when the replacement valve 100 is implanted. The atrial portion 104 may include a stent or other support structure that includes or is formed of a plurality of diamond shaped cells, although other suitable shapes, such as triangular, quadrilateral, or polygonal may be appropriate. In some examples, the atrial portion 104 may be formed as a braided mesh, as a unitary stent, or a combination thereof. According to one example, the atrial portion 104 may be laser cut from a tube of nitinol and heat set to a desired shape so that the atrial portion 104 is collapsible for delivery, and re-expandable to the set-shape during deployment. The atrial portion 104 may be heat set into a suitable shape to conform to the native anatomy of the valve annulus to help provide a seal and/or anchoring between the atrial portion 104 and the native valve annulus. The heat-set atrial portion 104 may be partially or entirely covered by a cuff or skirt, on the luminal and/or abluminal surface of the atrial portion 104. The skirt may be formed of any suitable material, including biomaterials such as bovine pericardium, biocompatible polymers such as ultra-high molecular weight polyethylene or combinations thereof. The atrial portion 104 may include features for connecting the atrial portion to a delivery system. For example, the atrial portion 104 may include pins or tabs around which sutures (or suture loops) of the delivery system may wrap.

As shown in Figure 1D, the atrial portion 104 may have two regions, a first region 126 with a disc shape and a second region 128 that is tapered. The disc shape of the atrial portion 104 may be configured to fit into and conform against the atrial side of the native valve annulus. The second region 128 of the atrial portion may be a strut region that supports prosthetic leaflets of the replacement valve 100. The strut region may be a portion of the stent forming the atrial portion 104 to which one or more prosthetic leaflets are coupled to form a prosthetic valve assembly, the prosthetic valve assembly configured to allow unidirectional flow of blood through the prosthetic valve assembly. For example, the first region 126 with the disc shape may have a first diameter in the expanded configuration and the second region 128, including leaflet support structure 112, may have a second diameter in the expanded configuration. The diameter of the second region 128 and leaflet support structure 112 in the expanded configuration may be smaller than the diameter of the first region 126. The atrial portion 104 may taper from the first region 126 to the second region 128.

The second region 128 of the atrial portion 104 may have a diameter in the expanded configuration that is smaller than the diameter of the central region 121 of the ventricular portion 102 when in the expanded configuration. For example, when the replacement valve 100 is expanded such that the ventricular portion 102 is on the ventricular side of the native valve annulus and the atrial portion 104 is on the atrial side of the native valve annulus, the second region 128 of the atrial portion 104 may be expanded and located radially within the central region 121 and third flared region 124 of the ventricular portion 102.

The leaflet support structure 112 may be integral with the second region 128 of atrial portion 104. However, in other examples, the leaflet support structure 112 is separate from and, therefore, not integral with the second region 128 of atrial portion 104. There may be a connection and, therefore, a connection region between the second region 128 and leaflet support structure 112. The connection and connection region may provide for isolation of the leaflet support structure from motion from the native anatomy, for example movement of the native valve annulus during contractions of the ventricles and the atria.

A separate leaflet support structure 112 may allow for the leaflet support structure 112 and second region 128 to have different wall thicknesses, different materials, different laser cut patterns, etc. By being able to have different properties between the leaflet support structure 112 and the second region 128, the overall design of the atrial portion 104 may be enhanced. For example, having a different wall thickness, different materials, or different laser cut patterns in both the leaflet support structure 112 and the second region 128, the overall stiffness of the atrial portion 104 may be adjusted. According to some examples, increasing the thickness of the walls of the second region 128 such that the second region is thicker than the walls of the leaflet support structure 112 may increase the overall stiffness of the atrial potion 104. In some examples, decreasing the thickness of the walls of the second region 128 such that the second region 128 is thinner than the thickness of the walls of the leaflet support structure 112 may decrease the overall stiffness of the atrial portion 104. For example, a softer, less radially stiff, atrial portion 104 may conform more to the native anatomy. In such an example, the softer, less radially stiff atrial portion may be easier to collapse and/or compress into a delivery system. According to some examples, a more rigid atrial portion 104 may provide support to the native anatomy. More support may prevent implant deflection and/or deformation. In such an example, a more rigid atrial portion 104 may prevent fatigue or decreased function of the atrial portion 104 and leaflet support portion.

As shown in Figure 1E, the atrial portion 104 may include a plurality of atrial anchoring points 114. The atrial anchoring points 114 may be located on the first region 126, second region 128, or at a location between the first region 126 and the second region 128 of the atrial portion 104, as shown in Figure 1D. For example, the atrial anchoring points 114 may be located midway between the proximal end and the distal end of the atrial portion 102. The proximal end refers to the end of the atrial portion 104 that is farthest away from the native valve annulus when the replacement valve 100 is positioned within the native valve annulus. The distal end refers to the end of the atrial portion 104 that is closest to the native valve annulus when the replacement valve 100 is positioned within the native valve annulus. In some examples, the atrial anchoring points 114 may be located closer to the proximal end or the distal end of the atrial portion 104. Thus, the location of the atrial anchoring points 114 shown in the figures is merely one example and is not meant to be limiting.

The atrial anchoring points 114 may be a plurality of holes for receiving the plurality of links 106. The plurality of links 106 may extend through corresponding ones of the plurality of atrial anchoring points 114. For example, as shown in Figure 1D, the links 106 may extend proximally from the ventricular portion 102 and through a respective atrial anchoring point 114 on the atrial portion 104. The links 106 may extend from the ventricular portion 102 and through the atrial anchoring points 114 during delivery of the replacement valve. For example, links 106 may tether or couple the ventricular portion 102 to atrial portion 104 during delivery. Links 106 may be adjusted in length, as described herein, once the replacement valve is deployed and positioned in its final implanted position. According to some examples, having separate atrial and ventricular portions 104, 102 with a flexible connection may allow the atrial and ventricular portions 104, 102 to be tuned more independently.

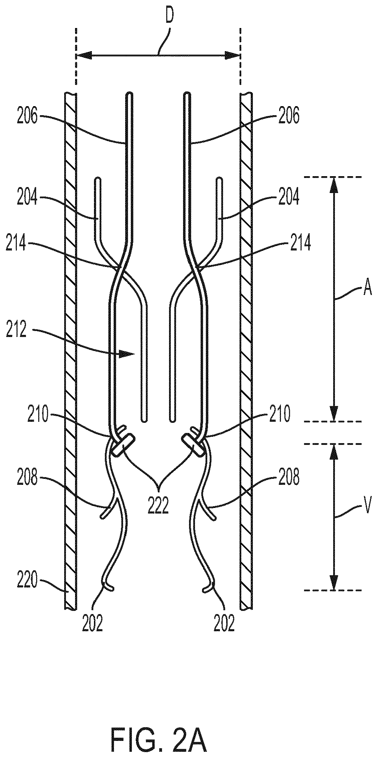

Figures 2A and 2B illustrate an example of a replacement valve of the present disclosure in a collapsed and expanded configuration, respectively. As shown in Figure 2A, the replacement valve may be placed into a delivery system, such as a within a delivery sheath 220, in the collapsed configuration. The ventricular portion 202 may have an axial length "V" when in the collapsed configuration and the atrial portion 204 may have an axial length "A" when in the collapsed configuration. As shown, the ventricular portion 202 and atrial portion 204 are shown as axially aligned, such that there may be some overlap of the ventricular portion 202 and atrial portion 204 in the radial direction. According to some examples, the ventricular portion 202 and atrial portion 204 may be axially offset to reduce the radial overlap of the ventricular portion 202 and the atrial portion 204, thereby reducing the profile or diameter of the replacement valve when in the collapsed configuration. The diameter "D" of the delivery sheath 220 may also be reduced based on the reduced profile and/or diameter of the replacement valve when in the collapsed configuration.

The overall axial length of the replacement valve when in the delivery sheath 220 may change based on the location of the atrial portion 204 with respect to the ventricular portion 202. For example, during delivery, the atrial portion 202 need not be affixed to the plurality of links 206 and, therefore, may change its position relative to the plurality of links 206 to be closer to the ventricular portion 202 or farther away from the ventricular portion 202. While only two links 206 are shown in Fig. 2A, the number of links in the figures is not meant to be limiting. Thus, there can be any number of links 206. For example, there may be 1, 2, 5, 8, 12, etc. links 206 as part of the replacement valve. Examples of suitable structures for the links are described in greater detail below.

As illustrated in Figure 2A, the plurality of links 206 may include a stop 222 at the distal end of each of the links 206 to prevent the links 206 from passing through the ventricular anchoring point 210 on the ventricular portion 202. Each link 206 may extend proximally through the delivery sheath 220 and through the plurality of atrial anchoring points 214 on the atrial portion 204. The plurality of links 206 may be positioned radially outward from the second region or leaflet support structure 212 of the atrial portion 204. The links 206 may continue proximally through the delivery sheath 220 and, ultimately, may be attached to a delivery device.

The stents or support structures of the replacement valves may be made of, or partially made of, a super elastic material such as nitinol. However, other biocompatible metals and metal alloys may be suitable. For example, super elastic and/or self-expanding metals other than nitinol may be suitable, while still other metals or metal alloys such as cobalt chromium or stainless steel may be suitable, particularly if the stent or support structure is intended to be balloon expandable. The replacement valves may be skirted to prevent leakage through and/or around the replacement valve. For example, one or more cuffs and/or skirts may be positioned on part or all of the interior/luminal surface of the stent of the replacement valve, and/or on part or all of the exterior/abluminal surface of the stent of the replacement valve. Such cuffs or skirts may be formed from any suitable biocompatible material, such as fabrics or polymers including ultra-high molecular weight polyethylene, biological materials such as bovine or porcine pericardium, or combinations thereof. In some embodiments the replacement valve is adapted to self-expand as it exits the delivery sheath. For example, it may completely self-expand, or in other embodiments it may partially self-expand and partially expand by non-self-expanding influences (e.g., a balloon), or it may be fully balloon-expandable.

Figure 2B illustrates an example replacement valve of the present disclosure in the expanded configuration. When in the expanded configuration, the atrial portion 204 may extend radially outward from a longitudinal axis of the replacement valve farther than does the ventricular portion 202. In some examples, the ventricular portion 102 may extend radially outward from the longitudinal axis of the replacement valve farther than does the atrial portion 204 when in the expanded configuration. The leaflet support structure 212 may be located radially inward of the ventricular portion 202 when the replacement valve is in the expanded configuration. The plurality of links 206 may be coupled to the ventricular portion 202 through the ventricular anchoring points 210. The plurality of links 206 may be braced from being pulled through the ventricular anchoring points 210 due to the stop 222 at the end of the links 206. For example, stops 222 may have a size, width, or diameter that is larger than the diameter or opening of the ventricular anchoring points. The stops 222 may abut or be in contact with the surface of the ventricular portion 202 opposite the side of the ventricular portion 202 in contact with the mitral valve annulus. Examples of suitable structures for the stops 222 are described in greater detail below.

The plurality of links 206 may extend proximally away from the ventricular portion 202 and through the atrial anchoring points 214 of the atrial portion 204. The atrial anchoring points 214 may be, for example, apertures through which a main portion of links 206 may slide through. The links 206 may continue to extend proximally and be coupled to a deployment mechanism of the delivery device. As is described below, excess material of link 206 may be removed after implantation of the replacement valve. As shown in Figure 2B, the links 206 may extend in a substantially straight line between ventricular anchoring point 210 and atrial anchoring point 214 and proximally to the delivery device (not shown). Links 206 may be made of a flexible material, such as a flexible metal, plastic, etc. Thus, links 206 may be bent, torqued, or configured such that links 206 are not substantially straight between ventricular anchoring point 210 and atrial anchoring point 214.

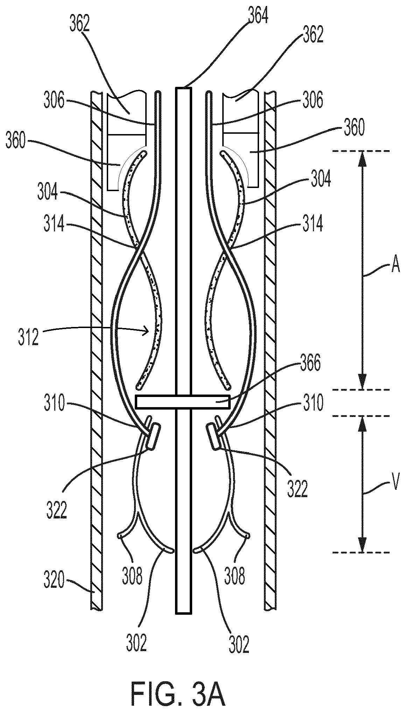

Figures 3A-3E are similar to Figures 2A and 2B in that they illustrate an example of a replacement valve of the present disclosure in a collapsed and expanded configuration, respectively. The shape of the ventricular portion 302 and atrial portion 304 may differ from ventricular portion 202 and atrial portion 204 but they may function in a similar fashion. While Figures 2A-3E show two different examples of the shapes of the ventricular and atrial portions, they are merely examples. The ventricular and atrial portions may vary in shape and size from the illustrated examples. The material of the atrial and ventricular portions 304, 302 may be the same or similar materials as described above in relation to atrial and ventricular portions 304, 302.

Figures 3A-3E illustrate a method of deploying the replacement valve. Figure 3A illustrates the replacement valve in a collapsed configuration within delivery sheath 320. The replacement valve may include a ventricular portion 302 having a plurality of retention features 308, such as barbs. The ventricular portion 302 may include a plurality of ventricular anchoring points 310 through which the plurality of links 306 may pass. The links 306 may include a stop 322 that prevents the links 306 from sliding through ventricular anchoring points 310. Thus, the stops 322 may abut against a luminal surface of the ventricular portion 302. The luminal surface refers to the surface of the ventricular portion 302 that does not abut the native valve annulus.

The atrial portion 304 is shown as axially aligned with the ventricular portion 302 such that there may be some radial overlap of the ventricular portion 302 and atrial portion 304. In some examples, the atrial portion 304 is spaced from the ventricular portion 302 when in the collapsed configuration within delivery sheath 320. For example, the proximal portion of the ventricular portion 302 may be spaced a distance from the distal portion of the atrial portion 304 when in the delivery sheath 320. Thus, the ventricular portion 302 and the atrial portion 304 may not radially overlap when in the collapsed configuration within the delivery sheath 320. The ventricular portion 302 may be tethered to the atrial portion 304 by a plurality of links 306. The plurality of links 306 may provide the atrial portion 304 and ventricular portion 302 the ability move with respect to one another while in the delivery sheath 320 such that the ventricular portion 302 and atrial portion 304 do not have to be axially aligned. By allowing the ventricular portion 302 and atrial portion 304 to be spaced from one another such that they do not overlap the overall profile or diameter of replacement valve in the collapsed configuration may be reduced, thereby reducing the diameter of the delivery mechanism and delivery sheath 320. Further, the diameter of the deployment mechanism of the delivery device and delivery sheath 320 may be reduced as the atrial portion 304 and ventricular portion 302 are free to move while in the delivery sheath 320. By way of example only, the atrial portion 304 and ventricular portion 302 may shift in and out of axial alignment with one another, or move closer or further apart while in the delivery sheath 320.

The atrial portion 304 may be located proximally to the ventricular portion 302 when in the delivery sheath 320. The links 306 may extend proximally from the ventricular portion 302 through atrial anchoring points 314 of the atrial portion 304. The links 306 may be located radially outward around leaflet support structure 312 such that the links 306 form a ring around the atrial portion 304.

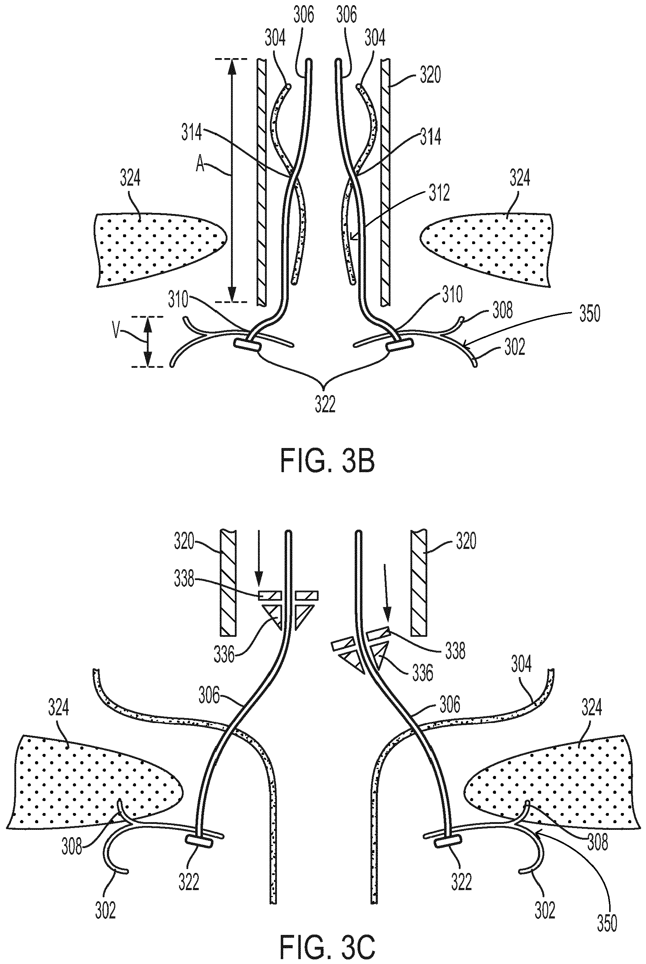

Figure 3B illustrates the ventricular portion 302 passing through the delivery sheath 320 and being deployed within a ventricle. The delivery sheath 320 may be located radially inward of the mitral valve annulus 324. The ventricular portion 302 may be advanced distally in the delivery sheath 320 such that the ventricular portion 302 exits the delivery sheath 320. In some examples, the delivery sheath 320 may be withdrawn. In such an example, the ventricular portion 302 may be braced against an internal layer of the delivery device or catheter. As shown in Figure 3A, internal layer 362 may be a dedicated layer for the purpose of bracing the ventricular and atrial portions 302, 304. The internal layer 362 may, in some examples, be used for steering the ventricular and atrial portions 302, 304. In some examples, there may be a ventricular platform 366. The ventricular platform 366 may be connected to a separate catheter layer 364.

Once the ventricular portion 302 exits the delivery sheath 320, the ventricular portion 302 may expand within the ventricle. For example, the ventricular portion 302 may self-expand once the ventricular portion 302 exits the delivery sheath 320. When the ventricular portion 302 exits the delivery sheath 320 and expands, the ventricular portion 302 may radially extend such that the diameter of the ventricular portion 302 in the expanded configuration is greater than the diameter of the ventricular portion 302 in the collapsed configuration. In some examples, the diameter of the ventricular portion 302 in the expanded configuration may be greater than the diameter of the delivery sheath 320.

The diameter of the ventricular portion 302 when in the expanded configuration may be large enough to allow for the retention features 308 to engage with the surface of the mitral valve annulus 324 facing the ventricle, and/or with native mitral valve leaflets. When the ventricular portion 302 is in the expanded configuration, the retention features 308 may extend from the abluminal surface 350 of the ventricular portion 302 such that the retention features 308 extend towards the mitral valve annulus 324. The retention features 308 may pierce tissue of the mitral valve annulus 324 in order to help maintain the ventricular portion 302 in a desired position and orientation, for example by resisting migration toward the atrium while there is relatively high pressure in the ventricle during ventricular systole. Similarly, the diameter of the ventricular portion 302 in the expanded condition may be larger than the diameter of the native mitral valve annulus 324, which may provide additional resistance to migration of the replacement valve toward the atrium, particularly during ventricular systole.

Figure 3C illustrates the atrial portion 304 deployed through the delivery sheath 320 and being exposed in the atrium. The atrial portion 304 may be advanced distally in the delivery sheath 320 such that the atrial portion exits the delivery sheath 320. As discussed with reference to Figure 3A, the delivery sheath 320 may be withdrawn, thereby deploying the atrial portion. In such an example, the atrial portion 304 may be braced against an internal layer of the delivery device or catheter. The atrial portions 302 may include platforms 360 for bracing. For example, platforms 360 may be countered to push the atrial portions 304 out of the delivery sheath 320 without catching on the delivery device. The contour of the platforms 360 may support the symmetry of the atrial portions 304 collapse. Once the atrial portion 304 exits the delivery sheath 320, the atrial portion 304 may expand within the atrium. For example, the atrial portion 304 may self-expand once the atrial portion exits the delivery sheath 320. When the atrial portion 304 exits the delivery sheath 320 and expands, the atrial portion 304 may radially extend such that the diameter of the atrial portion 304 in the expanded configuration is greater than the diameter of the atrial portion 304 in the collapsed configuration. It should be understood that, although much or most of atrial portion 304 expands into the atrium, at least a portion of the atrial portion 304, including the portion housing the prosthetic leaflets, may expand into the space within the native mitral valve annulus between the atrium and the ventricle.

At some time, whether before, contemporaneously, or after the atrial portion 304 exits the delivery sheath 320, the retention features 308 may engage with the mitral valve annulus 324. As shown in Figure 3C, the retention features 306 may become embedded or engaged with the mitral valve annulus 324 to secure the ventricular portion 302, and therefore the replacement valve, in its location in the ventricle.

Figure 3C illustrates an anchoring mechanism on each of the links 306. The anchoring mechanism may include a flexible locking mechanism 336. The flexible locking mechanism 336 is configured to advance distally along link 306. When the replacement valve is in the collapsed configuration, the flexible locking mechanism 336 is located proximally to the ventricular portion 302 and atrial portion 304. When the replacement valve is in the expanded configuration, the flexible locking mechanism 336 is located proximally to the luminal surface of the atrial portion 304 such that the flexible locking mechanism 336 can move distally towards the ventricular portion 302.

While not shown in Figure 3C, link 306 may include a plurality of locking features. The locking features may only be located on a portion or length of link 306. The locking feature may engage with flexible locking mechanism 336. For example, as flexible locking mechanism 336 passes over a locking feature, the flexible locking mechanism 336 may be locked into place until pushed further distally. The flexible locking mechanism 336 is configured such that once the flexible locking mechanism 336 advances distally over a locking feature, the flexible locking mechanism 336 cannot move proximally. Examples of suitable structures for the anchoring mechanism, including flexible locking mechanism 336 and locking features, are described in greater detail below.

Located proximally to the flexible locking mechanism 336 on link 306 is driver 338. The driver 338 may be used to push the flexible locking mechanism 336 distally along link 306 and over each of the locking features on link 306. A compression coil, hollow helical strand, or other lumen configuration may be used to actuate driver 338. In some examples, the compression coil may travel the full length of the delivery device. A wire may travel through the center of the compression coil and connect to the proximal end of link 306. The wire may be affixed to a feature in the handle of the delivery device such that the wire may not be advanced distally past a certain point or length. The compression coil may be affixed to a different mechanism within the delivery device or directly to a control input, such as in a handle of the delivery device. The activation of the mechanism or control input in the delivery device may push the compression coil distally within the handle of the delivery device and, therefore, may translate the force and/or motion of the compression coil through the catheter and into the distal region. The compression coil may impart force on the links without un-bending, or changing, the catheter's positioning. As the compression coil pushes the driver 338 distally, the wire may hold link 306 thereby allowing the driver 338 to push flexible locking mechanism 336 over each of the locking features. According to some examples, flexible locking mechanism 336 may be pushed over each of the locking features by the compression coil. Thus, flexible locking mechanism 336 may be advanced without using driver 338.

In some examples, there may be multiple inputs or controls to allow for individual tuning of each driver 338 on each of the respective links 306. In another example, there may be a single input or control with semi or fully consolidated internal actuation. The consolidated internal actuation system may be a pulley or similar force limiter to automatically provide even distribution of clamping force on the native anatomy during the deployment of the replacement valve.

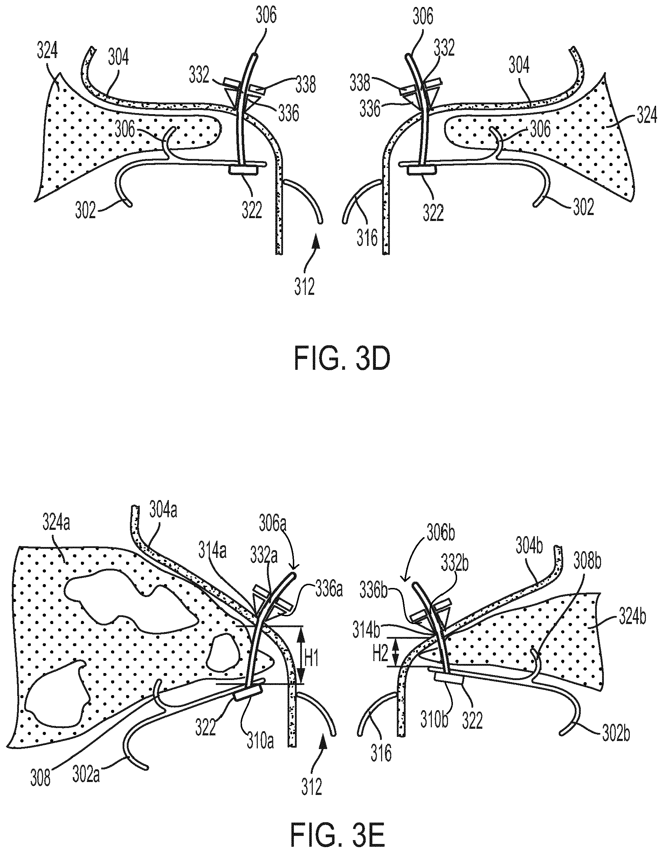

Figure 3D illustrates the replacement valve once the flexible locking mechanism 336 has been advanced distally. When the ventricular portion 302 and atrial portion 304 are in the collapsed configuration, the atrial portion 304 is a first distance from the ventricular portion 302. When the atrial portion 304 and the ventricular portion 302 are in the expanded configuration and the anchoring mechanism is not activated, i.e. the flexible locking mechanism 336 has not been advanced, the atrial portion 304 may be a second distance from the ventricular portion 302. The second distance may be less than the first distance. When the atrial portion 304 and the ventricular portion 302 are in the expanded configuration and the anchoring mechanism is activated, such that the flexible locking mechanism 336 is advanced distally, the atrial portion 304 may be a third distance from the ventricular portion 302. The third distance may be smaller than the second distance. Thus, the advancement of the flexible locking mechanism 336 may cause the atrial portion 304 to move closer to the ventricular portion 302.

The flexible locking mechanism 336 may be advanced distally towards the ventricular portion 302 using driver 338. The flexible locking mechanism 336 may advance distally on a portion 332 of link 306 that includes a plurality of locking features. The portion 332 of link 306 that includes the plurality of locking features may be a flexible length of the link 306 that includes teeth, spheres, or other features that enable a ratcheting motion that engages with flexible locking member 336. The portion 332 of link 306 with the locking features may my made of metal, plastic, or other biocompatible material so long as the portion 332 of link 306 is flexible.

As the flexible locking mechanism 336 is advanced distally towards the ventricular portion 302, the atrial portion 304 and ventricular portion 302 may be cinched together. For example, as the flexible locking mechanism 336 is advanced distally towards the ventricular anchor 302, the atrial anchor 304 may move distally due to the force of the flexible locking mechanism 336 on the atrial portion 304. As both the atrial portion 304 and flexible locking mechanism 336 are advanced distally, the tension on link 306 may increase. For example, stop 322 may brace link 306 against ventricular portion 302 for tensioning, thereby preventing link 322 from being pulled through the ventricular anchoring point as the flexible locking mechanism 336 is advanced distally. As the flexible locking mechanism 336 is advanced to secure the positioning of the replacement valve, the tension on each of the links 306 may increase. Once a desired tension is reached, thereby cinching the atrial portion 304 and the ventricular portion 302 against the native valve annulus, the portion 332 of link 306 proximal to the flexible locking mechanism 336 is severed, as described in greater detail below.

Each of the plurality of links 306 is tensioned in a similar fashion. Each of the plurality of links 306 may be tensioned individually at different times, simultaneously, or nearly simultaneously. Each link 306 may be tensioned to a different amount. According to some examples, each link 306 may have a different tension and/or a different length once fully tensioned. In some examples, two or more links 306 may have the same tension and/or length once fully tensioned. The delivery system may apply the same tensioning force to each of the plurality of links 306 without regard to the length of the link 306.

Figure 3E illustrates an example replacement valve being inserted into a native annulus that has different thicknesses on opposite sides. To adapt to the different size, shape, and/or thickness of the native annulus, each link 306 may be tensioned to a different amount and/or length. For example, one link 306a may be tensioned based on the thickness of a portion of native annulus 324a. Flexible locking mechanism 336a may be advanced distally over the portion 332a of link 306a that has the locking features such that link 306a is tensioned to have a distance or height H1 between ventricular anchoring point 310a of ventricular portion 302a and atrial anchoring point 314a of atrial portion 304a. The height H1 may correspond to the thickness of the native annulus 324a at the location of link 306a. Link 306a may not go through the annulus 324a. For example, link 306a may push the native annulus radially outward to provide space for link 306a. In other examples, link 306 may fit within the native valve opening such that link 306a does not push against the native annulus 324 but, instead, may fit radially inward of the native annulus 324a.

Flexible locking mechanism 336b may be advanced distally over the portion 332b of link 306b that has the locking features such that link 306b is tensioned to have a distance or height H2 between the ventricular anchoring point 310b of ventricular portion 302b and atrial anchoring point 314b of atrial portion 304b. The height H2 may correspond to the thickness of the native annulus 324b at the location of link 306b. While only two links 306 are described, each of the plurality of links may be tensioned to a different height or distance between the respective ventricular anchoring point 310 of ventricular portion 302 and atrial anchoring point 314 of atrial portion 304.

By allowing each link 306 to be tensioned differently, the replacement valve may be implanted in various patient anatomies. For example, cinching the ventricular portion and atrial portion together using an anchoring mechanism may provide for the treatment of extreme patient anatomies, where one side of the annulus has more deterioration than the other or where the thickness of the annulus is so minimal the ventricular portion and atrial portion must be implanted very close to one another to provide an adequate seal. By individually tensioning the plurality of links, the seal around the annulus of the patient may be optimized resulting in a more effective implant. Moreover, allowing for each of the plurality of links to be tensioned individually to different tensions and/or lengths, the replacement valve becomes more patient specific as the resulting implanted replacement valve is tensioned based on the patient's anatomy.

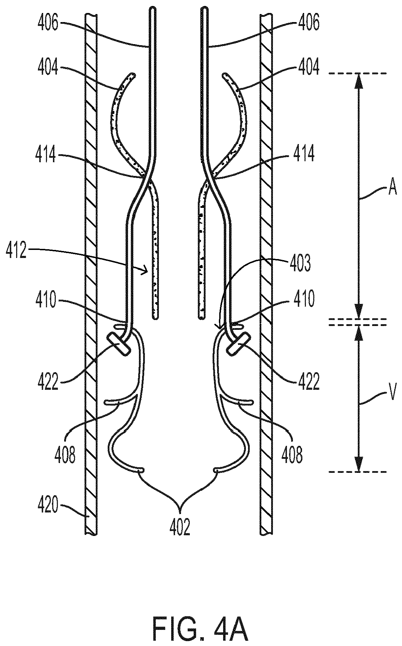

Figures 4A and 4B are similar to Figures 2A-2B and 3A-3E in that they illustrate an example of a replacement valve in collapsed and expanded configurations, respectively. Thus, the numbering of the elements for the replacement valve illustrated in Figures 4A and 4B substantially correspond to the numbering of elements in Figures 2A-3E. The shape of ventricular portion 402 and atrial portion 404 may differ from ventricular portion 202, 302 and atrial portion 204, 304 but they may function in a similar fashion.

Figure 4A illustrates the replacement valve in the collapsed configuration within a delivery sheath. The ventricular portion 402 may have an axial length "V" when in the collapsed configuration and the atrial portion 404 may have an axial length "A" when in the collapsed configuration. The ventricular portion 402 and atrial portion 404 may be axially aligned when in the delivery sheath 420, such that there may be some overlap of the ventricular portion 402 and atrial portion 404 in the radial direction. According to some examples, the ventricular portion 402 and atrial portion 404 may be axially offset when within the delivery sheath 420. In such an example, allowing the ventricular portion 402 and atrial portion 404 to shift axially with respect to one another may reduce the radial overlap of the ventricular portion 402 and the atrial portion 404, thereby reducing the profile or diameter of the replacement valve when in the collapsed configuration.

The overall length of the replacement valve when in the delivery sheath 420 may change based on the location of the atrial portion 404 on the plurality of links 406 with respect to the ventricular portion 402. During delivery, the atrial portion 404 may change its position or location on the plurality of links 406 to be closer to the ventricular portion 402 or further away from the ventricular portion 402.

The plurality of links 406 may include a stop 422 at the distal end of each of the links 406 to prevent the links 406 from passing through the ventricular anchoring point 410 on the ventricular portion 402. Examples of suitable structures for the links 406 and stops 422 are described in greater detail below. The ventricular anchoring points 410 may be located along curvature 403 of ventricular portion 402. The stop 422 may brace the link 406 for tensioning, as will be described herein. Each link 406 may extend proximally through the delivery sheath 420 and through the plurality of atrial anchoring points 414 on the atrial portion 404. The plurality of links 406 may be positioned on the atrial portion 404 radially outward from the second region or leaflet support structure 412. The links 406 may continue proximally through the delivery sheath 420 and, ultimately, may be attached to a delivery device

Figure 4B illustrates the replacement valve of Figure 4A in an expanded configuration. When in the expanded configuration, stop 422 may be located between two parts or portions of ventricular portion 402. For example, due to curvature 403 of ventricular portion 402, stop 422 may be located in area 405. In some examples, the atrial portion 404 may extend radially outward farther than the ventricular portion 402. The leaflet support structure 412 may be located radially inward of the ventricular portion 402 when the replacement valve is in the expanded configuration. The plurality of links 406 may extend through ventricular anchoring points 410. The plurality of links 406 may not be pulled through the ventricular anchoring points 410 due to the stop 422 at the distal end of the links 406.

The plurality of links 406 may extend proximally away from the ventricular portion and through the atrial anchoring points 414 of the atrial portion 404. The links 406 may continue to extend proximally and be coupled to the delivery mechanism. As will be described herein, the excess link 406 material may be removed after implantation of the replacement valve. As shown in Figure 4B, the links 406 may extend in a substantially straight line between ventricular anchoring point 410 and atrial anchoring point 414 and proximally to the delivery device (not shown). Links 406 may be made of a flexible material, such as a flexible metal, plastics, etc. Thus, links 406 may be bent, torqued, or configured such that links 406 are not substantially straight between ventricular anchoring point 410 and atrial anchoring point 414.

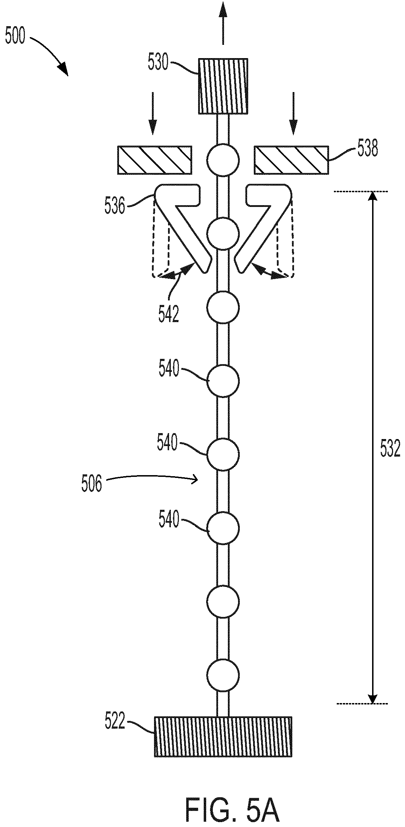

Figures 5A-5D illustrate an example of an anchoring mechanism. The anchoring mechanism 500 described herein with respect to Figures 5A-5D may be used with any of the replacement valves described above. The anchoring mechanism 500 may use or employ a beaded security type ratcheting mechanism. Each of the plurality of links 506 may include an anchoring mechanism 500. As shown in Figure 5A, the anchoring mechanism 500 may include a delivery system retention feature 530, a driver 538, and a flexible locking mechanism 536.

Each of the plurality of links 506 may be attached or coupled to a delivery system retention feature 530. The delivery system retention feature 530 may be attached to, or be a part of, the delivery system such that the delivery system retention feature 530 is deployed after the replacement valve exits the delivery sheath and expands.

The driver 538 may be used to advance the flexible locking member 536 distally, towards stop 522. The driver 538 may be actuated by a compression coil, hollow helical strand, or other lumen configuration, as described in detail above.

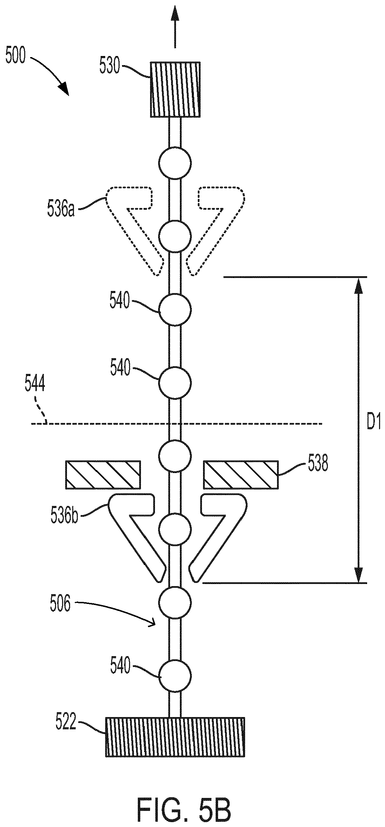

The flexible locking mechanism 536 is configured to engage with each of the locking features 540. The locking features 540 may extend along a portion 532 of link 506. The locking features 540 may be teeth, spherical or substantially spherical features, or other features that enable a ratcheting type of motion. As shown in Figures 5A and 5B, the locking features 540 are spherical or substantially spherical features. The locking features 540 may have fixed positions relative to links 506. As the flexible locking mechanism 536 advances distally over each of the locking features 540, the flexible locking mechanism 536 may bend or flex outwardly 542 to advance onto or over each of the locking features 540. The flexible locking mechanism 536 is configured such that it may only move distally over each of the locking features 540 but may not move proximally after the distal advancement. Thus, the flexible locking mechanism 536 may only cinch the ventricular portion and atrial portion closer together. In some embodiments, the flexible locking mechanism 536 may include a release such that if a link 506 is over tensioned the flexible locking mechanism 536 may be able to move proximally thereby decreasing the tension. The release may allow for finer tuning or corrections during tensioning of the links. The flexible locking mechanism 536 may include a sub-mechanism. For example, the sub-mechanism (not shown) may include a wedge to wedge the flexible locking mechanism 536 open or, in some example, pull the locking mechanism open.

Figure 5B illustrates the anchoring mechanism of Figure 5A once the flexible locking mechanism has been advanced distally. Once the diver 538 and flexible locking mechanism 536 are advanced distally to provide the proper tensioning of link 506, the excess link 506 proximal to the driver 538 may be severed at a location 544 on link 506. For example, flexible locking mechanism 536 may be advanced distally a distance D1 from a starting point or location on link 506 shown by flexible locking mechanism 536a. Once link 506 is properly tensioned, flexible locking mechanism 536 may be at a point or location on link 506 shown by flexible locking mechanism 536b. Link 506 may be severed at location 544, located proximally to driver 538. For example, link 506 may be severed at a location between driver 538 and delivery system retention feature 530. In some examples, the link 506 may be severed just proximal to driver 538. Severing link 506 may occur after the replacement valve has been deployed and the flexible locking mechanism 536b advanced distally such that the atrial portion and ventricular portion are cinched.

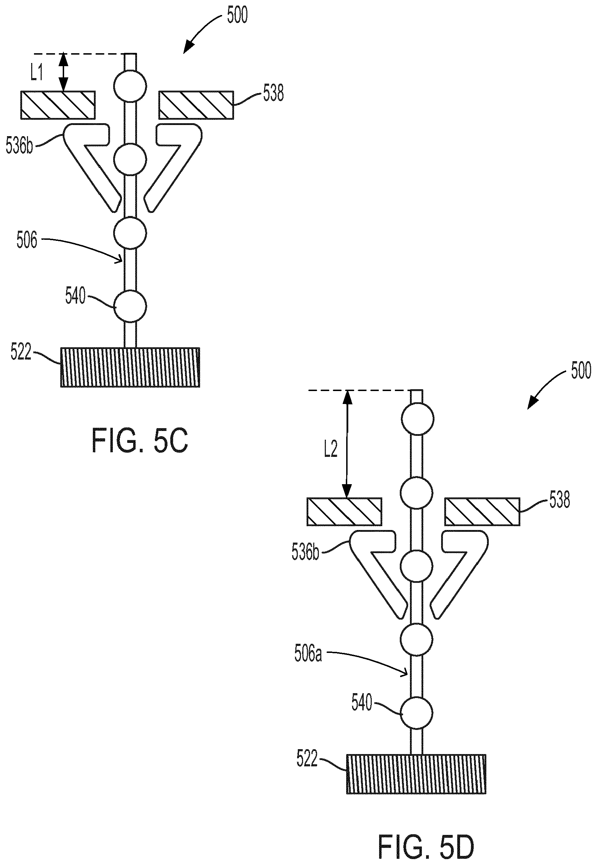

To sever the link 506, the link 506 may be melted using a resistance wire segment. In some examples, the link 506 may be unscrewed at a predetermined length once the driver 538 is advanced beyond a certain point on link 506. In this example, unscrewing link 506 may result in a variable length tail. The tail is the portion of link 506 proximal to driver 538 once link 506 is unscrewed. For example, the tail of link 506 in Figure 5C may have a shorter length L1 as compared to the tail of link 506a in Figure 5D. Unscrewing link 506 may allow for a smaller delivery sheath and delivery system at the detachment mechanism is small and simple. In yet another example, the link may be cut with a razor mechanism. The razor mechanism may be part of the delivery system and housed just proximal of driver 538.

Figures 5C and 5D illustrate the link having a variable length once it is severed. As illustrated in Figure 5C, once link 506 is severed, the excess, non-tethered portion of link 506 proximal to flexible locking mechanism 536 is removed. This may leave a tail portion with length L1. As illustrated in Figure 5D, link 506a may have a tail portion with length L2. Length L2 of link 506a may be greater than length L1 of link 506. In some examples, length L2 of link 506a may be less than length L1 of link 506. Thus, links 506, 506a may have a variable length once severed. The excess link 506, 506a may remain coupled to delivery system retention feature 530 and ultimately removed by the delivery system.

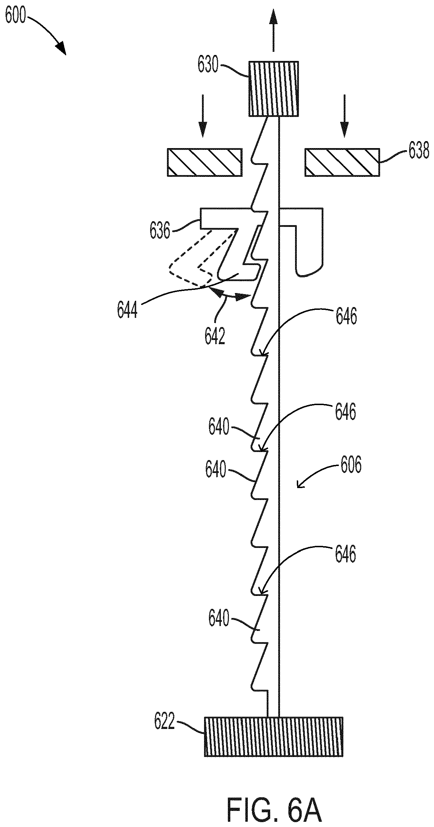

Figures 6A-6D illustrate an example of another type of anchoring mechanism. The anchoring mechanism 600 described herein with respect to Figures 6A-6D may be used with any of the replacement valves described above. The anchoring mechanism 600 may function similarly to anchoring mechanism 500 and, therefore, the numbering of elements for anchoring mechanism illustrated in Figures 6A-6D substantially correspond to the numbering of elements in Figures 5A-5D. The anchoring mechanism 600 may use or employ a zip-tie like ratcheting mechanism. Each of the plurality of links 606 may include an anchoring mechanism 600. As shown in Figure 6A, the anchoring mechanism 600 may include a delivery system retention feature 630, a driver 638, and a flexible locking mechanism 636.

Link 606 may have one surface or face that is substantially planar and a second surface with a plurality of locking features 640 opposite the substantially planar surface. The plurality of locking features 640 may be similar to teeth or ridges along the link 606. The locking features 640 may be configured to allow flexible locking mechanism 636 to move distally past each locking feature 640 but it may not allow the flexible locking mechanism to move proximally. For example, flexible locking mechanism 636 may have a portion 644 that engages with the ledge 646 of locking feature 640. If flexible locking mechanism were to be pulled proximally along link 606, portion 644 may be stopped by ledge 646 thereby preventing proximal movement of flexible locking mechanism 636. Thus, the flexible locking mechanism 636 may only allow ventricular portion and atrial portion to move closer to one another and not farther away.

Driver 638 may be used to advance flexible locking member 636. As a force is exerted on driver 638, driver 638 may exert a force on flexible locking mechanism 636. As the driver 638 exerts a force on flexible locking mechanism 636, flexible locking mechanism 636 may flex or bend 642 outwardly as flexible locking mechanism 636 advances over locking features 640.

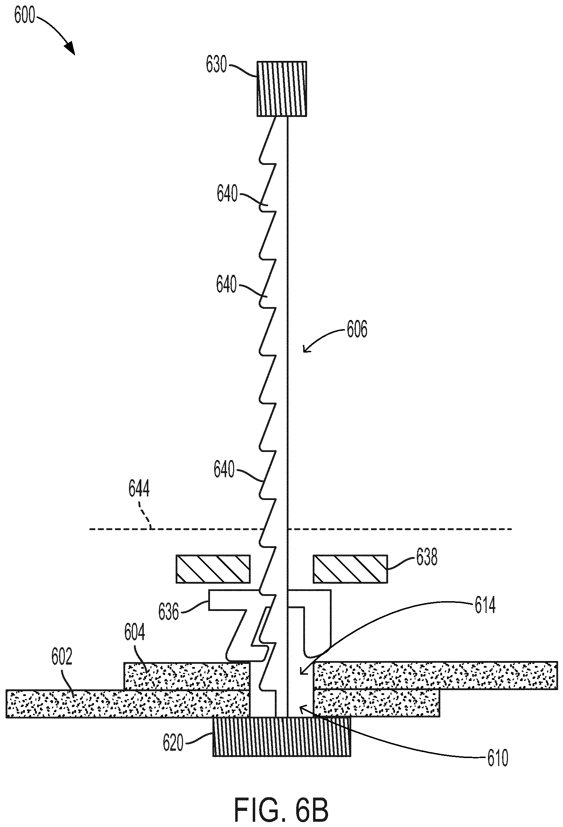

Figure 6B illustrates the anchoring mechanism of Figure 6A once the flexible locking mechanism 636 has been advanced distally. Flexible locking mechanism 636 may be advanced distally such that ventricular portion 602 at the ventricular anchoring point 610 abut atrial portion 604 at atrial anchoring point 614. Thus, there may be no distance between ventricular portion 602 and atrial portion 604 at that link 606. When there is no distance between ventricular portion 602 and atrial portion 604 there may be maximum tension on link 606. In some examples, there may be a larger distance between ventricular portion 602 at ventricular anchoring point 610 and atrial portion 604 at atrial anchoring point 614. The distance between ventricular portion 602 at ventricular anchoring point 610 and atrial portion 604 at atrial anchoring point 614 may be based on the native annulus and the necessary tension to provide the optimum seal.

Once link 606 is tensioned, the excess link 606 may be severed. For example, the excess link material may be any portion of link 606 that is proximal to driver 638, as noted by severance line 644. The severance line 648 may be at any point along link 638 between driver 638 and delivery system retention feature 630.

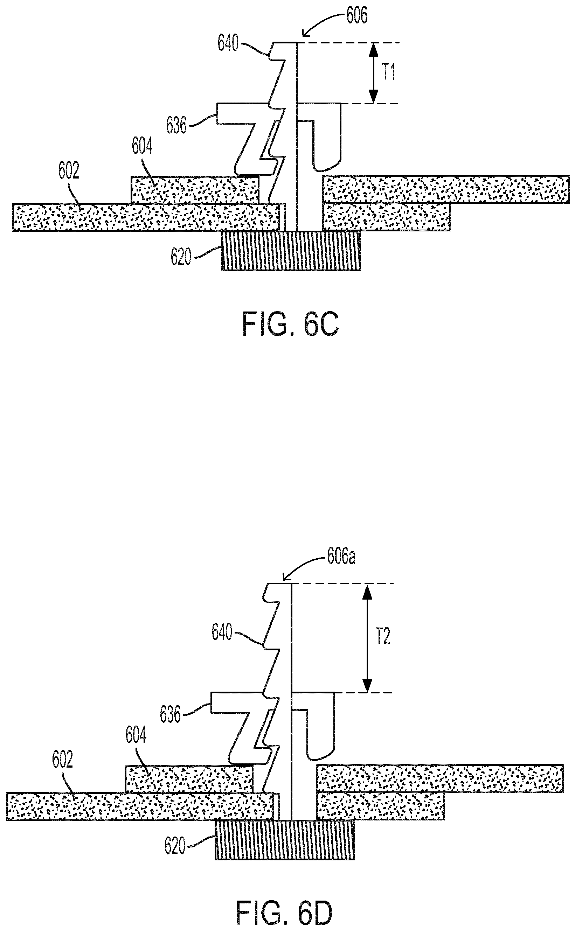

As shown in Figures 6C and 6D, the severed link may be a variable length. As illustrated in Figure 6C, link 606 may be severed leaving a tail portion with length T1. As illustrated in Figure 6D, link 606a may be severed leaving a tail portion with length T2. Length T2 of link 606a may be greater than length T1 of link 606. In some examples, length T2 may be less than length T1. Once link 606, 606a is severed, the excess, non-tethered portion of link 606, 606a proximal to flexible locking mechanism 636 is removed. The excess link 606, 606a may remain coupled to delivery system retention feature 630 and ultimately removed by the delivery system.

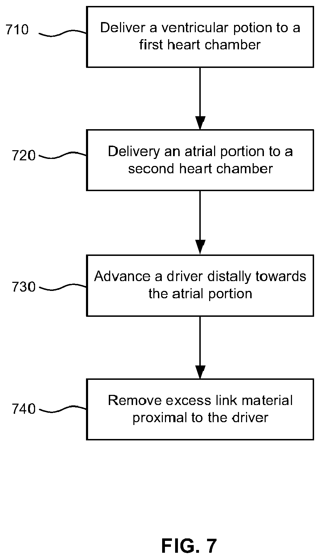

Figure 7 illustrates an example method for implanting the replacement valve. For example, in block 710, the ventricular portion may be delivered to a first heart chamber. To deliver the ventricular portion to the first heart chamber, the replacement valve may be in a collapsed configuration within a delivery sheath. The replacement valve may be advanced distally through the delivery sheath such that the ventricular portions exits the delivery sheath into the first heart chamber on a first side of a mitral valve annulus. As the ventricular portion exits the delivery sheath, the ventricular portion may expand from a collapsed configuration to an expanded configuration. The ventricular portion may self-expand upon exiting the delivery sheath.

In block 720, the atrial portion may be delivered to a second heart chamber on a second side of the mitral valve annulus. The atrial portion may be tethered or linked to ventricular portion via a plurality of links. Thus, as the ventricular portion advanced distally, the atrial portion may advance distally. The atrial portion may have to advance further distally once the ventricular portion exits the delivery sheath in order for the atrial portion to exit the delivery sheath. As the atrial portion exits the delivery sheath, the ventricular portion may expand from a collapsed configuration to an expanded configuration.

In block 730, a driver may be advanced distally towards the atrial portion. The driver may be part of an anchoring mechanism. The anchoring mechanism may include a flexible locking mechanism that is configure to engage with the plurality of locking features along each of the links. As the driver is advanced distally, the driver may exert a force on the flexible locking mechanism thereby causing the flexible locking mechanism to advance distally. As the driver and flexible locking mechanism advance distally, the atrial portion and ventricular portion may be cinched together, thereby creating a seal around the mitral valve annulus. Each of the plurality of links may include a respective anchoring mechanism and, therefore, each of the respective anchoring mechanisms may be adjusted individually allowing for a patient specific fit.

In block 740, the excess link material may be removed. After the drive and flexible locking mechanism are advanced distally, thereby cinching together the atrial and ventricular portions, the excess material located proximal to the drive may be severed and removed.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A replacement cardiac valve comprising:
an atrial portion configured to expand from a collapsed configuration to an expanded configuration;
a ventricular portion separate from the atrial portion and configured to expand from a collapsed configuration to an expanded configuration, the ventricular portion having a first plurality of anchoring points; and
a plurality of links, each of the plurality of links coupled to a respective one of the first plurality of anchoring points;
wherein at least one of the atrial portion or the ventricular portion is moveable with respect to the plurality of links to adjust a distance between the atrial portion and the ventricular portion when the atrial portion and the ventricular portion are each in the expanded configuration.

2. The replacement cardiac valve of claim 1, wherein the ventricular portion further includes a plurality of barbs extending from an abluminal surface of the ventricular portion, the plurality of barbs configured to engage with a ventricular side of a native valve annulus when the ventricular portion is in the expanded configuration.

3. The replacement cardiac valve of claim 1 or claim 2, wherein the atrial portion includes a second plurality of anchoring points, each of the plurality of links extending through a respective one of the second plurality of anchoring points.

4. The replacement cardiac valve of any one of the preceding claims, wherein the atrial portion includes a leaflet support.

5. The replacement cardiac valve of claim 4, wherein the leaflet support is integral with the atrial portion.

6. The replacement cardiac valve of claim 4 or claim 5, wherein the leaflet support includes at least one replacement leaflet coupled to the leaflet support.

7. The replacement cardiac valve of any one of claims 4 to 6, wherein the atrial portion has a first diameter and the leaflet support has a second diameter smaller than the first diameter when each of the atrial portion and the leaflet support are in the expanded configuration, where the first diameter tapers to the second diameter.

8. The replacement cardiac valve of any one of the preceding claims, wherein an outflow end of the atrial portion is axially spaced from an inflow end of the ventricular portion when in a collapsed configuration.

9. The replacement cardiac valve of any one of the preceding claims, wherein when the atrial portion and the ventricular portion are in the collapsed configuration the atrial portion is a first distance from the ventricular portion, when the atrial portion and the ventricular portion are in the expanded configuration and an anchoring mechanism is not activated the atrial portion is a second distance from the ventricular portion, the second distance being smaller than the first distance, and when the atrial portion and the ventricular portion are in the expanded configuration and the anchoring mechanism is activated, the atrial portion is a third distance from the ventricular portion, the third distance being smaller than the second distance.

10. The replacement cardiac valve of any one of the preceding claims, further comprising an anchoring mechanism having a flexible locking mechanism located proximally to a luminal surface of the atrial portion, the flexible locking mechanism configured to move distally towards the ventricular portion.

11. The replacement cardiac valve of claim 10, wherein each of the plurality of links further includes a flexible locking mechanism and a plurality of locking features, each flexible locking mechanism configured to engage with the plurality of locking features.

12. The replacement cardiac valve of claim 10 or claim 11, wherein the anchoring mechanism is a ratcheting mechanism.

13. The replacement cardiac valve of any one of claims 10 to 12, wherein the anchoring mechanism further includes a driver located proximal to the flexible locking mechanism, the driver configured to move the flexible locking mechanism distally towards the ventricular anchor.

14. The replacement cardiac valve of any one of the preceding claims, wherein the plurality of links extend between the first plurality of anchoring points and a delivery system.
